# EUROPÄISCHE PATENTSCHRIFT

(11) **EP 1 069 101 B1**
(45) Veröffentlichungstag und Bekanntmachung des Hinweises auf die Patenterteilung: **01.10.2003**
(21) Anmeldenummer: 00114104.3
(22) Anmeldetag: 10.07.2000
(51) Int. Cl.: C07C 11/10, C07C 11/107, C07C 11/06, C07C 7/00, C07C 7/148, C07C 7/177, C07C 7/10

(54) **Verfahren zur Herstellung von C5-/C6-Olefinen**
Process for the preparation of C5-/C6-olefins
Procédé pour la préparation d'oléfines en C5-/C6

(30) Priorität: 12.07.1999 DE 19932060
(43) Veröffentlichungstag der Anmeldung: 17.01.2001
(73) Patentinhaber: BASF AKTIENGESELLSCHAFT, 67056 Ludwigshafen (DE)
(72) Erfinder: Schwab, Peter, Dr., 67098 Bad Dürkheim (DE); Schulz, Michael, Dr., 67067 Ludwigshafen (DE); Schulz, Ralf, Dr., 67346 Speyer (DE); Huber, Sylvia, Dr., 64673 Zwingenberg (DE)
(74) Vertreter: Isenbruck, Günter, Dr.

(56) Entgegenhaltungen:
- EP-A- 0 945 415
- DE-A- 19 746 040
- US-A- 3 590 096

## Beschreibung

Die vorliegende Erfindung betrifft ein Verfahren zur Umwandlung von olefinischen C₄-Kohlenwasserstoffen, beispielsweise aus Steamcrackern oder FCC-Crackem, in Pentene und Hexene mittels Metathesereaktion. Als gewünschtes Verfahrens-Koppelprodukt wird Propen erhalten.

Die Olefinmetathese (Disproportionierung) beschreibt in ihrer einfachsten Form die reversible, metallkatalysierte Umalkylidenierung von Olefinen durch Bruch oder Neuformierung von C=C-Doppelbindungen gemäß nachfolgender Gleichung:

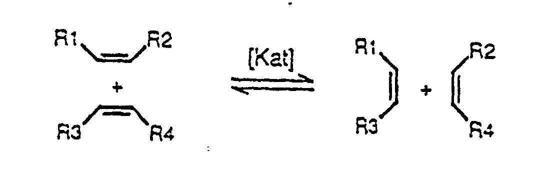

Im speziellen Fall der Metathese von acyclischen Olefinen unterscheidet man zwischen Selbstmetathese, bei der ein Olefin in ein Gemisch zweier Olefine unterschiedlicher molarer Masse übergeht (beispielsweise: Propen → Ethen + 2-Buten), und Kreuz- oder Co-Metathese, die eine Reaktion zweier unterschiedlicher Olefine beschreibt (Propen + 1-Buten → Ethen + 2-Penten). Ist einer der Reaktionspartner Ethen, so spricht man im allgemeinen von einer Ethenolyse.

Als Metathesekatalysatoren eignen sich prinzipiell homogene und heterogene Übergangsmetall-Verbindungen, insbesondere die der VI. bis VIII. Nebengruppe des Periodensystems der Elemente sowie homogene und heterogene Katalysatorsysteme, in denen diese Verbindungen enthalten sind.

Unterschiedliche Metathese-Verfahren, die von C₄-Strömen ausgehen, sind beschrieben.

US 5,057,638 betrifft ein Verfahren zur Herstellung von 1-Hexen, umfassend die Verfahrensschritte:
a) Metathese von 1-Buten zu einer Mischung aus 3-Hexen und Ethen,
b) Abtrennen des 3-Hexens aus dem in Schritt a) gewonnenen Produktgemisch
c) Umsetzung des 3-Hexens mit einem reaktiven Wasserstoff, vorzugsweise aus Wasser oder Carbonsäure stammend, enthaltenden Elektrophil unter sauren Bedingungen, welche die Addition der elektrophilen Komponenten an die C=C-Bindung erlauben (z.B. Hydratisierung),
d) Cracken des Produkts aus Schritt c), z.B. durch Dehydratisierung, zur Herstellung eines Gemisches aus n-Hexenen, welches 1-Hexen in wirtschaftlich akzeptablen Mengen enthält.

US 3 595 920 (**05.05.1969**) (Gulf Res. & Dev.Co.) beschreibt die Umwandlung von kurzkettigen C₄-C₁₂-Olefinen (vorzugsweise α-Olefinen) in höhere Olefine durch Metathese. Das Verfahren ist dadurch gekennzeichnet, daß das Ausgangsolefin mit einem Katalysator, welcher Aluminium, Molybdän oder Rhenium und Silber oder Kupfer bei 100 bis 240°C enthält, in Kontakt gebracht wird, wobei leichtersiedende Nebenprodukte, wie z.B. Ethen, in situ aus dem Gleichgewicht entfernt werden können.

Die vorliegende Erfindung betrifft ferner ein kombiniertes Verfahren zur Herstellung von C₅/C₆-Olefinen mit Propen als Nebenausbeute ausgehend von C₄-Schnitten von Steam- oder FCC-Crackern.

Steamcracker stellen die Hauptquelle für petrochemische Basischemikalien, wie Ethen, Propen, C₄-Olefine und höhere Kohlenwasserstoffe dar. Beim Crackprozeß ist es notwendig, große Energiemengen bei hohen Temperaturen in einer Zeitspanne zu übertragen, die einerseits zwar ausreicht, die Spaltung durchzuführen, andererseits aber eine Weiterreaktion der Spaltprodukte nicht zuläßt. Bei der Spaltung von Kohlenwasserstoffen wird die Ausbeute an Ethen und Propen daher im wesentlichen durch die
- Art der eingesetzten Kohlenwasserstoffe (Naphta, Ethan, LPG, Gasöl, o.ä.)
- Spalttemperatur,
- Verweilzeit
- und die Partialdrücke der jeweiligen Kohlenwasserstoffe
bestimmt.

Die höchste Ausbeute an Ethen und Propen wird bei Spalttemperaturen zwischen 800 und 850°C und Verweilzeiten von 0,2 bis 0,5 s erzielt. Hauptprodukt ist in diesem Bereich stets Ethen, wobei das C₃/C₂-Austragsverhältnis von ca. 0,5 bis 0,7 durch Variation der Crack-Bedingungen in geringem Ausmaß erhöht werden kann. Weltweit steigt der Bedarf an Propen rascher an als der von Ethen. Dies hat u.a. zur Folge, daß Verfahren zur Downstream-Verwertung der höheren, beim Crackprozeß gebildeten Kohlenwasserstoffe, wie z.B. C₄, in Hinblick auf die Optimierung der Propenausbeute stark an Bedeutung gewinnen.

Im Rahmen von Arbeiten zur Verbesserung der Wertschöpfung von Steamcracker-Nebenprodukten stellte sich die Aufgabe, ein flexibel steuerbares katalytisches Verfahren zur Gewinnung reiner C₅-/C₆-Olefinströme aus kostengünstig verfügbaren olefinhaltigen C₄-Kohlenwasserstoffgemischen ohne Ethen-Zudosierung zu entwickeln.

Die Aufgabe wird erfindungsgemäß gelöst durch ein Verfahren zur Herstellung von C₅-/C₆-Olefinen aus einem olefinischen, C₄-Kohlenwasserstoffe enthaltenden Ausgangsstrom, wobei
a) in Gegenwart eines Metathesekatalysators der mindestens eine Verbindung eines Metalls der VI.b, VII.b oder VIII. Nebengruppe des Periodensystems der Elemente enthält, eine Metathesereaktion durchgeführt wird, im Rahmen derer im Ausgangsstrom enthaltener 1-Buten, 2-Buten und Isobuten zu einem Gemisch aus C₂-C₆-Olefinen und Butanen umgesetzt werden,
b) der so erhaltene Austragsstrom zunächst destillativ getrennt wird in eine aus C₂-C₄-Olefinen und Butanen bestehende Leichtsiederfraktion **A**, die ausgeschleust wird, sowie in eine aus C₄-C₆-Olefinen und Butanen bestehende Schwersiederfraktion,
c) die aus b) erhaltene Schwersiederfraktion anschließend destillativ in eine aus Butenen und Butanen bestehende Leichtsiederfraktion **B**, eine aus Penten und Methylbuten bestehende Mittelsiederfraktion **C** und in eine aus Hexen und Methylpenten bestehende Schwersiederfraktion **D** getrennt wird,
d) wobei die Fraktionen **B** und/oder **C** komplett oder teilweise in den Verfahrensschritt a) zurückgeführt werden und die Fraktion **D** als Produkt ausgeschleust wird.

Der Ausdruck _{"}bestehend aus" schließt dabei nicht die Gegenwart kleinerer Mengen anderer Kohlenwasserstoffe aus.

Dabei wird in einstufiger Reaktionsführung gemäß nachfolgender Gleichung in einer Metathesereaktion eine aus C₄-Olefinen, vorzugsweise n-Butenen, Isobuten und Butanen bestehende Fraktion an einem homogenen oder vorzugsweise heterogenen Metathesekatalysator zu einem Produktgemisch aus (inerten) Butanen, nicht umgesetztem 1-Buten, 2-Buten und gegebenenfalls Isobuten sowie den Metatheseprodukten Ethen, Propen, 2-Penten, gegebenenfalls 2-Methyl-2-Buten, 3-Hexen und gegebenenfalls 2-Methyl-2-Penten umgesetzt:

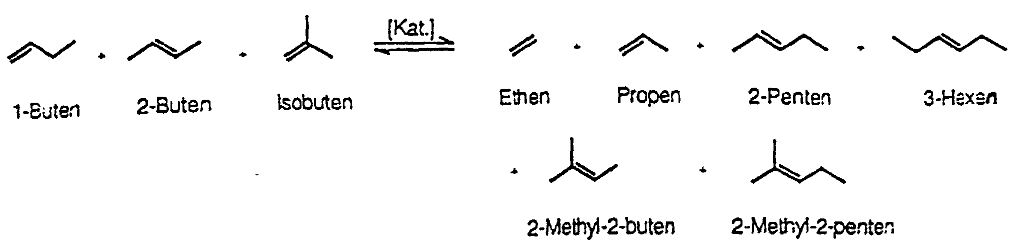

Die Menge an den verzweigten C₅- und C₆-Kohlenwasserstoffen im Metatheseaustrag ist abhängig vom Isobuten-Gehalt im C₄-Feed und wird vorzugsweise möglichst gering (<3 %) gehalten.

Um das erfindungsgemäße Verfahren in mehreren Variationen näher zu erläutern, wird die obige Gleichgewichtsreaktion (ohne Berücksichtigung von Isobuten) in drei wichtige Einzelreaktionen unterteilt:

### 1. Kreuzmetathese von 1-Buten mit 2-Buten

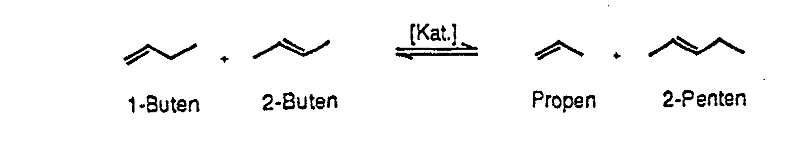

### 2. Selbstmetathese von 1-Buten

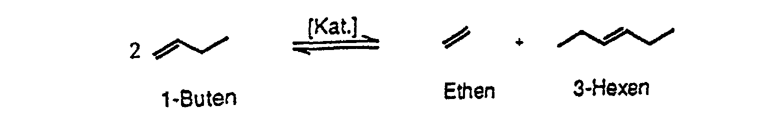

### 3. Ethenolyse von 2-Buten

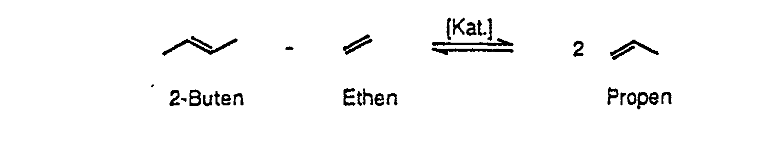

In Abhängigkeit vom jeweiligen Bedarf an den Zielprodukten Propen, 2-Penten und 3-Hexen (die Bezeichnung 2-Penten beinhaltet unter anderem eventuell gebildete Isomere, wie cis/trans oder 2-Methyl-2-Buten, entsprechendes gilt für 3-Hexen) kann die äußere Massenbilanz des Verfahrens gezielt durch Verschiebung des Gleichgewichts durch Rückführung bestimmter Teilströme beeinflußt werden. So wird beispielsweise die 3-Hexenausbeute erhöht, wenn durch Rückführung von 2-Penten in den Metatheseschritt die Kreuzmetathese von 1-Buten mit 2-Buten unterdrückt wird, so daß hier kein oder möglichst wenig 1-Buten verbraucht wird. Bei der dann bevorzugt ablaufenden Selbstmetathese von 1-Buten zu 3-Hexen wird zusätzlich Ethen gebildet, welches in einer Folgereaktion mit 2-Buten zum Wertprodukt Propen reagiert.

Olefingemische, die 1-Buten, 2-Buten und Isobuten enthalten, werden u.a. bei diversen Crackprozessen wie Steamcracking oder FCC-Cracking als C₄-Fraktion erhalten. Alternativ können Butengemische, wie sie bei der Dehydrierung von Butanen oder durch Dimerisierung von Ethen anfallen, eingesetzt werden. In der C₄-Fraktion enthaltene Butane verhalten sich inert. Diene, Alkine oder Enine werden vor dem erfindungsgemäßen Metatheseschritt mit gängigen Methoden wie Extraktion oder Selektivhydrierung entfernt.

Der Butengehalt der im Verfahren eingesetzten C₄-Fraktion beträgt 1 bis 100 Gew.-%, vorzugsweise 60 bis 90 Gew.-%. Der Butengehalt bezieht sich dabei auf 1-Buten, 2-Buten und Isobuten.

Vorzugsweise wird eine C₄-Fraktion eingesetzt, wie sie beim Steam- oder FCC-Cracken oder bei der Dehydrierung von Butan anfällt.

Dabei kann als C₄-Fraktion Raffinat I oder II eingesetzt werden, wobei der C₄-Strom vor der Metathese-Reaktion durch entsprechende Behandlung an Adsorber-Schutzbetten, bevorzugt an hochoberflächigen Aluminiumoxiden oder Molsieben von störenden Verunreinigungen befreit wird.

Die erhaltene Leichtsiederfraktion A, insbesondere die C_{2/3}-Fraktion, kann direkt als solche weiterverarbeitet werden, der Aufarbeitungssequenz eines Steam- oder FCC-Crackers zugeführt werden, um reines Ethen und Propen zu gewinnen, oder ganz oder teilweise in den Metatheseschritt zurückgeführt werden, um die Ausbeute an Penten/Hexen zu erhöhen; oder separat zur Gewinnung von Ethen und Propen als Reinkomponente verwendet werden (insbesondere als C_{2/3}-Fraktion).

Die Metathesereaktion wird dabei vorzugsweise in Gegenwart von heterogenen, nicht oder nur geringfügig isomerisierungsaktiven Metathesekatalysatoren durchgeführt, die aus der Klasse der auf anorganischen Trägern aufgebrachten Übergangsmetallverbindungen von Metallen der VI.b, VII.b oder VIII.-Gruppe des Periodensystems der Elemente ausgewählt sind.

Bevorzugt wird als Metathesekatalysator Rheniumoxid auf einem Träger, vorzugsweise auf γ-Aluminiumoxid oder auf Al₂O₃/B₂O₃/SiO₂-Mischträgern eingesetzt.

Insbesondere wird als Katalysator Re₂O₇/γ-Al₂O₃ mit einem Rheniumoxid-Gehalt von 1 bis 20 %, vorzugsweise 3 bis 15 %, besonders bevorzugt 6 bis 12 % (Gew.-%) eingesetzt.

Die Metathese wird bei Flüssigfahrweise vorzugsweise bei einer Temperatur von 0 bis 150°C, besonders bevorzugt 20 bis 80°C sowie einem Druck von 2 bis 200 bar, besonders bevorzugt 5 bis 30 bar, durchgeführt.

Wenn die Metathese in der Gasphase durchgeführt wird, beträgt die Temperatur vorzugsweise 20 bis 300°C, besonders bevorzugt 50 bis 200°C. Der Druck beträgt in diesem Fall vorzugsweise 1 bis 20 bar, besonders bevorzugt 1 bis 5 bar.

Im Rahmen von Arbeiten zur Verbesserung der Wertschöpfung von Steamcracker-Nebenprodukten stellte sich zudem die Aufgabe, eine flexibel steuerbare Verfahrenssequenz zur Verwertung von C₄-Schnitt zu entwickeln. Ziel war es, C₄-Olefine mit hoher Wertschöpfung in höherpreisige Olefinfraktionen umzuwandeln. Als Einsatzstoff steht Roh-C₄-Schnitt aus Steamcrackern oder FCC-Crackern zur Verfügung.

Die Aufgabe wird erfindungsgemäß gelöst durch ein Verfahren zur Herstellung von C₅/C₆-Olefinen und Propen aus Steamcracker- oder Raffinerie-C₄-Strömen, umfassend die Teilschritte
(1) Abtrennung von Butadien und acetylenischen Verbindungen durch gegebenenfalls Extraktion von Butadien mit einem Butadien-selektiven Lösungsmittel und nachfolgend oder alternativ Selektivhydrierung von in Roh-C₄-Schnitt enthaltenen Butadienen und acetylenischen Verunreinigungen um einen Reaktionsaustrag zu erhalten, der n-Butene und Isobuten und im wesentlichen keine Butadiene und acetylenischen Verbindungen enthält,
(2) Abtrennung von Isobuten durch Umsetzung des in der vorstehenden Stufe erhaltenen Reaktionsaustrags mit einem Alkohol in Gegenwart eines sauren Katalysators zu einem Ether, Abtrennung des Ethers und des Alkohols, die gleichzeitig oder nach der Veretherung erfolgen kann, um einen Reaktionsaustrag zu erhalten, der n-Butene und gegebenenfalls Oxygenat-Verunreinigungen enthält, wobei gebildeter Ether ausgetragen oder zur Reingewinnung von Isobuten rückgespalten werden kann und dem Veretherungsschritt ein Destillationsschritt zur Abtrennung von Isobuten nachfolgen kann, wobei gegebenenfalls auch eingeschleuste C₃-, i-C₄- sowie C₅-Kohlenwasserstoffe destillativ im Rahmen der Aufarbeitung des Ethers abgetrennt werden können, oder Oligomerisierung oder Polymerisation von Isobuten aus dem in der vorstehenden Stufe erhaltenen Reaktionsaustrag in Gegenwart eines sauren Katalysators, dessen Säurestärke zur selektiven Abtrennung von Isobuten als Oligo- oder Polyisobuten geeignet ist, um einen Strom zu erhalten, der 0 bis 15 % Rest-Isobuten aufweist,
(3) Abtrennen der Oxygenat-Verunreinigungen des Austrags der vorstehenden Schritte an entsprechend ausgewählten Adsorbermaterialien,
(4) Metathesereaktion des so erhaltenen Raffinat II-Stromes wie beschrieben.

Vorzugsweise wird der Teilschritt Selektivhydrierung von in Roh-C₄-Schnitt enthaltenen Butadien und acetylenischen Verunreinigungen zweistufig durchgeführt durch Inkontaktbringen des Roh-C₄-Schnittes in flüssiger Phase mit einem Katalysator, der mindestens ein Metall, ausgewählt aus der Gruppe Nickel, Palladium und Platin, auf einem Träger enthält, vorzugsweise Palladium auf Aluminiumoxid, bei einer Temperatur von 20 bis 200°C, einem Druck von 1 bis 50 bar, einer Volumengeschwindigkeit von 0,5 bis 30 m³ Frischfeed pro m³ Katalysator pro Stunde und einem Verhältnis von Recycle zu Zustrom von 0 bis 30 mit einem Molverhältnis von Wasserstoff zu Diolefinen von 0,5 bis 50, um einen Reaktionsaustrag zu erhalten, in welchem neben Isobuten die n-Butene 1-Buten und 2-Buten in einem Molverhältnis von 2:1 bis 1:10, vorzugsweise von 2:1 bis 1:2, vorliegen und im wesentlichen keine Diolefine und acetylenischen Verbindungen enthalten sind.

Vorzugsweise wird der Teilschritt Butadien-Extraktion aus Roh-C₄-Schnitt mit einem Butadien-selektiven Lösungsmittel durchgeführt, ausgewählt aus der Klasse polar-aprotischer Lösungsmittel, wie Aceton, Furfural, Acetonitril, Dimethylacetamid, Dimethylformamid und N-Methylpyrrolidon, um einen Reaktionsaustrag zu erhalten, in welchem die n-Butene 1-Buten und 2-Buten in einem Molverhältnis 2:1 bis 1:10, vorzugsweise von 2:1 bis 1:2, vorliegen.

Vorzugsweise wird der Teilschritt Isobuten-Veretherung in einer dreistufigen Reaktorkaskade mit Methanol oder Isobutanol, vorzugsweise Isobutanol in Gegenwart eines sauren Ionentauschers durchgeführt, in der geflutete Festbettkatalysatoren von oben nach unten durchströmt werden, wobei die Reaktor-Eingangstemperatur 0 bis 60°C, vorzugweise 10 bis 50°C, die Ausgangstemperatur 25 bis 85°C, vorzugsweise 35 bis 75°C, der Druck 2 bis 50 bar, vorzugsweise 3 bis 20 bar und das Verhältnis von Isobutanol zu Isobuten 0,8 bis 2,0, vorzugsweise 1,0 bis 1,5 beträgt sowie der Gesamtumsatz dem Gleichgewichtsumsatz entspricht.

Vorzugsweise wird der Teilschritt Isobuten-Abtrennung durch Oligomerisierung oder Polymerisation von Isobuten ausgehend von dem nach den vorstehend beschriebenen Stufen Butadien-Extraktion und/oder Selektivhydrierung erhaltenen Reaktionsaustrag in Gegenwart eines Katalysators durchgeführt, der ausgewählt ist aus der Klasse homogener und heterogener Broensted-Säuren, bevorzugt aus heterogenen Kontakten, die ein Oxid eines Metalls der VI.b Nebengruppe des Periodensystems der Elemente auch bei einem sauren anorganischen Träger enthalten, besonders bevorzugt WO₃TiO₂, um so einen Strom zu erzeugen, der weniger als 15 % Isobuten-Restgehalt aufweist.

### Selektivhydrierung von Roh-C₄-Schnitt

Alkine, Alkinene und Alkadiene sind aufgrund ihrer Neigung zur Polymerisation oder ihrer ausgeprägten Neigung zur Komplexbildung an Übergangsmetallen in vielen technischen Synthesen unerwünschte Stoffe. Sie beeinträchtigen die bei diesen Reaktionen verwendeten Katalysatoren zum Teil sehr stark.

Der C₄-Strom eines Steamcrackers enthält einen hohen Anteil mehrfach ungesättigter Verbindungen wie 1,3-Butadien, 1-Butin (Ethylacetylen) und Butenin (Vinylacetylen). Je nach vorhandener Downstream-Verarbeitung werden die mehrfach ungesättigten Verbindungen entweder extrahiert (Butadien-Extraktion) oder selektiv hydriert. Im erstgenannten Fall beträgt der Restgehalt mehrfach ungesättigter Verbindungen typischerweise 0,05 bis 0,3 Gew.-%, im letztgenannten Fall typischerweise 0,1 bis 4,0 Gew.-%. Da die Restmengen an mehrfach ungesättigten Verbindungen ebenfalls bei der Weiterverarbeitung stören, ist eine weitere Anreicherung durch Selektivhydrierung auf Werte < 10 ppm erforderlich. Um einen möglichst hohen Wertproduktanteil an Butenen zu erhalten, ist die Überhydrierung zu Butanen so gering wie möglich zu halten.

Geeignete Hydrierungskatalysatoren sind beschrieben:
- **J.P.Boitiaux, J.Cosyns, M.Derrien and G.Lèger, Hydrocarbon Processing, March 1985, p.51-59**
   Beschreibung bimetallischer Katalysatoren für Selektivhydrierungen von C₂-, C₃-, C₄-, C₅- und C₅₊-Kohlenwasserstoffströmen. Besonders bimetallische Katalysatoren aus Gp.VIII und Gp.IB-Metallen zeigen im Vergleich zu reinen Pd-Trägerkatalysatoren Verbesserungen in der Selektivität.
- **DE-A-2 059 978**
   Selektive Hydrierung von ungesättigten Kohlenwasserstoffen in flüssiger Phase an einem Pd/Tonerde-Katalysator. Der Katalysator ist dadurch gekennzeichnet, daß der Tonerde-Träger mit BET 120 m²/g zunächst einer Wasserdampf-Behandlung bei 110-300°C unterzogen wird und anschließend bei 500-1200°C calziniert wird. Zuletzt wird die Pd-Verbindung aufgebracht und bei 300-600°C calziniert.
- **EP-A-0 564 328 und EP-A-0 564 329**
   Katalysator, welcher u.a. aus Pd und In oder Ga auf Träger besteht. Die Katalysatorkombination ermöglicht einen Einsatz ohne CO-Zusatz bei hoher Aktivität und Selektivität.
- **EP-A-0 089 252**
   Pd, Au-Trägerkatalysatoren. Die Katalysatorherstellung umfaßt folgende Schritte:
   - Tränkung eines mineralischen Trägers mit einer Pd-Verbindung
   - Calzinierung unter O₂-haltigem Gas
   - Behandlung mit einem Reduktionsmittel
   - Tränkung mit einer halogenierten Au-Verbindung
   - Behandlung mit einem Reduktionsmittel
   - Auswaschen des Halogens durch basische Verbindung
   - Calzinierung unter O₂-haltigem Gas.
- **US 5,475,173**
   Katalysator bestehend aus Pd und Ag und Alkalifluorid auf anorganischem Träger.
   Vorteile des Katalysators: Durch KF-Zusatz erhöhter Butadien-Umsatz und bessere Selektivität zu Butenen (d.h. geringere Überhydrierung zu n-Butan).
- **EP-A-0 653 243**
   Katalysator zeichnet sich dadurch aus, daß sich die Aktivkomponente überwiegend in den Meso- und Makroporen befindet. Der Katalysator zeichnet sich weiterhin durch ein großes Porenvolumen und niedriges Rüttelgewicht aus. So besitzt der Katalysator aus Beispiel 1 ein Rüttelgewicht von 383 g/l und ein Porenvolumen von 1,17 ml/g.
- **EP-A-0 211 381**
   Katalysator aus Gp.VIII-Metall (vorzugsweise Pt) und mindestens ein Metall aus Pb, Sn oder Zn auf anorganischem Träger. Der bevorzugte Katalysator besteht aus Pt/ZnAl₂O₄. Durch die genannten Promotoren Pb, Sn und Zn wird die Selektivität des Pt-Kontaktes verbessert.
- **EP-A-0 722 776**
   Katalysator aus Pd und mindestens einem Alkalifluorid und optional Ag auf anorganischen Träger (Al₂O₃, TiO₂ und/oder ZrO₂). Die Katalysatorkombination ermöglicht eine Selektivhydrierung in Gegenwart von Schwefel-Verbindungen.
- **EP-A-0 576 828**
   Katalysator auf Basis von Edelmetall und/oder Edelmetaloxid auf Al₂O₃-Träger mit definiertem Röntgenbeugungsmuster. Der Träger besteht dabei aus n-Al₂O₃ und/oder γ-Al₂O₃. Der Katalysator besitzt aufgrund des speziellen Trägers eine hohe Anfangsselektivität und kann daher sofort zur selektiven Hydrierung ungesättigter Verbindungen eingesetzt werden. '
- **JP 01110594**
   Pd-Trägerkatalysator
   Zusätzlich wird ein weiterer Elektronendonor eingesetzt. Dieser besteht entweder aus einem auf dem Katalysator abgeschiedenem Metall, wie beispielsweise Na, K, Ag, Cu, Ga, In, Cr, Mo oder La, oder einem Zusatz zum Kohlenwasserstoff-Einsatzstoff, wie beispielsweise Alkohol, Ether oder N-haltige Verbindungen. Durch die genannten Maßnahmen kann eine Herabsetzung der 1-Buten-Isomerisierung erreicht werden.
- **DE-A-31 19 850**
   Katalysator aus einem SiO₂- oder Al₂O₃-Träger mit 10 bis 200 m²/g bzw.≤ 100 m²/g mit Pd und Ag als Aktivkomponente. Der Katalysator dient vornehmlich zur Hydrierung butadienarmer Kohlenwasserstoffströme.
- **EP-A-0 780 155**
   Katalysator aus Pd und einem Gp. IB-Metall auf einem Al₂O₃-Träger, wobei mindestens 80 % des Pd und 80 % des Gp. IB-Metalls in einer äußeren Schale zwichen r₁ (= Radius des Pellets) und 0,8- r₁ aufgebracht sind.

### Alternativ: Extraktion von Butadien aus Roh-C₄-Schnitt

Das bevorzugte Verfahren zur Butadien-Isolierung basiert auf dem physikalischen Prinzip der Extraktivdestillation. Durch Zusatz selektiver organischer Lösungsmittel wird die Flüchtigkeit spezieller Komponenten eines Gemisches, in diesem Fall Butadien, erniedrigt. Diese bleiben daher mit dem Lösungsmittel im Sumpf der Destillationskolonne, während die destillativ zuvor nicht abtrennbaren Begleitsubstanzen über Kopf entfernt werden können. Als Lösungsmittel für die Extraktivdestillation kommen hauptsächlich Aceton, Furfural, Acetonitril, Dimethylacetamid, Dimethylformamid (DMF) und N-Methylpyrrolidon (NMP) zur Anwendung. Extraktivdestillationen eignen sich besonders für butadienreiche C₄-Crackschnitte mit einem relativ hohen Anteil an Alkinen, u.a. Methyl-, Ethylund Vinylacetylen sowie Methylallen.

Das vereinfachte Prinzip einer Lösungsmittel-Extraktion aus Roh-C₄-Schnitt kann wie folgt dargestellt werden: Der vollständig verdampfte C₄-Schnitt wird einer Extraktionskolonne am unteren Ende zugeführt. Das Lösungsmittel (DMF, NMP) fließt von oben dem Gasgemisch entgegen und belädt sich auf dem Weg nach unten mit besserlöslichem Butadien und geringen Mengen an Butenen. Am unteren Ende der Extraktionskolonne wird ein Teil des gewonnenen Rein-Butadiens zugeführt, um die Butene weitestgehend auszutreiben. Die Butene verlassen die Trennsäule am Kopf. In einer als Ausgaser bezeichneten weiteren Kolonne wird das Butadien durch Auskochen vom Lösungsmittel befreit und anschließend reindestilliert.

Üblicherweise wird der Reaktionsaustrag einer Butadien-Extraktivdestillation in die zweite Stufe einer Selektivhydrierung eingespeist, um den Butadien-Restgehalt auf Werte von < 10 ppm zu reduzieren.

Der nach Abtrennung von Butadien verbleibende C₄-Strom wird als C₄-Raffinat oder Raffinat I bezeichnet und enthält in der Hauptsache die Komponenten Isobuten, 1-Buten, 2-Butene sowie n- und Isobutane.

### Abtrennung von Isobuten aus Raffinat I

Bei der weiteren Auftrennung des C₄-Stromes wird nachfolgend vorzugsweise Isobuten isoliert, da es sich durch seine Verzweigung und seine höhere Reaktivität von den übrigen C₄-Komponenten unterscheidet. Neben der Möglichkeit einer formselektiven Molsiebtrennung, mit welcher Isobuten mit einer Reinheit von 99 % gewonnen werden kann und an den Molsiebporen adsorbierte n-Butene und Butan mittels eines höhersiedenden Kohlenwasserstoffs wieder desorbiert werden können, geschieht dies in erster Linie destillativ unter Verwendung eines sog. Deisobutenizers, mit welchem Isobuten gemeinsam mit 1-Buten und Isobuten über Kopf abgetrennt wird und 2-Butene sowie n-Butan incl. Restmengen an Isound 1-Buten im Sumpf verbleiben, oder extraktiv durch Umsetzung von Isobuten mit Alkoholen an sauren Ionenaustauschern. Hierzu werden vorzugsweise Methanol (→ MTBE) oder Isobutanol (IBTBE) eingesetzt.

Die Herstellung von MTBE aus Methanol und Isobuten erfolgt bei 30 bis 100°C und leichtem Überdruck in der Flüssigphase an sauren Ionenaustauschern. Man arbeitet entweder in zwei Reaktoren oder in einem zweistufigen Schachtreaktor, um einen nahezu vollständigen Isobuten-Umsatz (> 99 %) zu erzielen. Die druckabhängige Azeotropbildung zwischen Methanol und MTBE erfordert zur Reindarstellung von MTBE eine mehrstufige Druckdestillation oder wird nach neuerer Technologie durch Methanol-Adsorption an Adsorberharzen erreicht. Alle anderen Komponenten der C₄-Fraktion bleiben unverändert. Da geringe Anteile von Diolefinen und Acetylenen durch Polymerbildung eine Verkürzung der Lebensdauer des Ionenaustauschers bewirken können, werden vorzugsweise bifunktionelle PD-enthaltende Ionenaustauscher eingesetzt, bei denen in Gegenwart kleiner Mengen Wasserstoff nur Diolefine und Acetylene hydriert werden. Die Veretherung des Isobutens bleibt hiervon unbeeinflußt.

MTBE dient in erster Linie zur Octanzahl-Erhöhung von Fahrbenzin. MTBE und IBTBE können alternativ an sauren Oxiden in der Gasphase bei 150 bis 300°C zur Reingewinnung von Isobuten rückgespalten werden.

Eine weitere Möglichkeit zur Abtrennung von Isobuten aus Raffinat I besteht in der direkten Synthese von Oligo/Polyisobuten. An sauren homogenen und heterogen Katalysatoren, wie z.B. Wolframtrioxid auf Titandioxid, kann auf diese Weise bei Isobuten-Umsätzen bis 95 % ein Austragsstrom erhalten werden, der über einen Restanteil an Isobuten von maximal 5 % verfügt.

### Feedreinigung des Raffinat II-Stroms an Adsorbermaterialien

Zur Verbesserung der Standzeit der eingesetzten Katalysatoren für den nachfolgenden Metatheseschritt ist wie vorstehend beschrieben, der Einsatz einer Feed-Reinigung (guard bed) zur Abtrennung von Katalysatorgiften, wie beispielsweise Wasser, Oxygenates, Schwefel oder Schwefelverbindungen bzw. organischen Halogeniden erforderlich.

Verfahren zur Adsorption und adsorptiven Reinigung sind beispielsweise beschrieben in W. Kast, Adsorption aus der Gasphase, VCH, Weinheim (1988). Der Einsatz von zeolithischen Adsorbentien wird erläutert bei D.W. Breck, Zeolite Molecular Sieves, Wiley, New York (1974).

Die Entfernung von speziell Acetaldehyd aus C₃ bis C₁₅-Kohlenwasserstoffen in flüssiger Phase kann gemäß EP-A-0 582 901 erfolgen.

### Selektivhydrierung von Roh-C₄-Schnitt

Aus der aus einem Steamcracker oder einer Raffinerie stammenden Roh-C₄-Fraktion wird zunächst Butadien (1,2- und 1,3-Butadien) sowie im C₄-Schnitt enthaltene Alkine oder Alkenine in einem zweistufigen Verfahren selektivhydriert. Der aus der Raffinerie stammende C₄-Strom kann gemäß einer Ausführungsform auch direkt in den zweiten Schritt der Selektivhydrierung eingespeist werden.

Der erste Schritt der Hydrierung wird vorzugsweise an einem Katalysator durchgeführt, der 0,1 bis 0,5 Gew.-% Palladium auf Aluminiumoxid als Träger enthält. Die Umsetzung wird in Gas/Flüssigphase im Festbett (Rieselfahrweise) mit einem Flüssigkreislauf betrieben. Die Hydrierung erfolgt bei einer Temperatur im Bereich 40 bis 80°C und einem Druck von 10 bis 30 bar, einem Molverhältnis von Wasserstoff zu Butadien von 10 bis 50 und einer Volumengeschwindigkeit LHSV von bis 15 m³ Frischfeed pro m³ Katalysator pro Stunde und einem Verhältnis von Recycle von Zustrom von 5 bis 20 betrieben.

Der zweite Schritt der Hydrierung wird vorzugsweise an einem Katalysator durchgeführt, der 0,1 bis 0,5 Gew.-% Palladium auf Aluminiumoxid als Träger enthält. Die Umsetzung wird in Gas/Flüssigphase im Festbett (Rieselfahrweise) mit einem Flüssigkreislauf betrieben. Die Hydrierung erfolgt bei einer Temperatur im Bereich von 50 bis 90°C und einem Druck von 10 bis 30 bar, einem Molverhältnis von Wasserstoff zu Butadien von 1,0 bis 10 und einer Volumengeschwindigkeit LHSV von 5 bis 20 m³ Frischfeed pro m³ Katalysator pro Stunde und einem Verhältnis von Recycle zu Zustrom von 0 bis 15 betrieben.

Die Hydrierung wird unter sog. "low isom"-Bedingungen durchgeführt, unter denen es zu keiner oder zumindest möglichst geringer C=C-Isomerisierung von 1-Buten zu 2-Buten kommt Der Restgehalt an Butadien kann je nach Hydrierschärfe 0 bis 50 ppm betragen.

Der so erhaltene Reaktionsaustrag wird als Raffinat I bezeichnet und weist neben Isobuten 1-Buten und 2-Buten in einem Molverhältnis von 2:1 bis 1:10, vorzugsweise von 2:1 bis 1:2 auf.

### Alternativ: Abtrennung von Butadien aus Roh-C₄-Schnitt via Extraktion

Die Extraktion von Butadien aus Roh-C₄-Schnitt erfolgt nach BASF-Technologie unter Verwendung von N-Methylpyrrolidon.

Der Reaktionsaustrag der Extraktion wird gemäß einer Ausführungsform der Erfindung in den zweiten Schritt der vorangehend beschriebenen Selektivhydrierung eingespeist, um Restmengen Butadien zu entfernen, wobei darauf zu achten ist, daß es zu keiner oder nur geringfügiger Isomerisierung von 1-Buten zu 2-Buten kommt.

### Abtrennung von Isobuten via Veretherung mit Alkoholen

In der Veretherungsstufe wird Isobuten mit Alkoholen, vorzugsweise mit Isobutanol, an einem sauren Katalysator, vorzugsweise an einem sauren Ionenaustauscher, zu Ether, vorzugsweise Isobutyl-tert.-butylether umgesetzt. Die Umsetzung erfolgt gemäß einer Ausführungsform der Erfindung in einer dreistufigen Reaktorkaskade, in der geflutete Festbettkatalysatoren von oben nach unten durchströmt werden. Im ersten Reaktor beträgt die Eingangstemperatur 0 bis 60°C, vorzugsweise 10 bis 50°C; die Ausgangstemperatur liegt zwischen 25 und 85°C, vorzugsweise zwischen 35 und 75°C, und der Druck beträgt 2 bis 50 bar, vorzugsweise 3 bis 20 bar. Bei einem Verhältnis von Isobutanol zu Isobuten von 0,8 bis 2,0, vorzugsweise 1,0 bis 1,5 beträgt der Umsatz zwischen 70 und 90 %.

Im zweiten Reaktor beträgt die Eingangstemperatur 0 bis 60°C, vorzugsweise 10 bis 50°C; die Ausgangstemperatur liegt zwischen 25 und 85, vorzugsweise zwischen 35 und 75°C, und der Druck beträgt 2 bis 50 bar, vorzugsweise 3 bis 20 bar. Der Gesamtumsatz über die zwei Stufen erhöht sich auf 85 bis 99 %, vorzugsweise 90 bis 97 %.

Im dritten und größten Reaktor wird bei gleicher Eingangs- und Ausgangstemperatur von 0 bis 60°C, vorzugsweise 10 bis 50°C; der Gleichgewichtsumsatz erzielt. An die Veretherung und Abtrennung des gebildeten Ethers schließt sich die Etherspaltung an: Die endotherme Reaktion wird an sauren Katalysatoren, vorzugsweise an sauren Heterogenkontakten, beispielsweise Phosphorsäure auf einem SiO₂-Träger, bei einer Eingangstemperatur von 150 bis 300°C, vorzugsweise bei 200 bis 250°C, und einer Ausgangstemperatur von 100 bis 250°C, vorzugsweise bei 130 bis 220°C durchgeführt.

Bei Einsatz von FCC-C₄-Schnitt ist damit zu rechnen, daß Propan in Mengen um 1 Gew.-%, Isobuten in Mengen um 30 bis 40 Gew.-% sowie C₅-Kohlenwasserstoffe in Mengen um 3 bis 10 % eingeschleust werden, welche die nachfolgende Verfahrenssequenz beeinträchtigen können. Im Rahmen der Aufarbeitung des Ethers ist demzufolge die Möglichkeit einer destillativen Abtrennung der genannten Komponenten vorgesehen.

Der so erhaltene, als Raffinat II bezeichnete Reaktionsaustrag weist einen Isobuten-Restgehalt von 0,1 bis 3 Gew.-% auf.

Bei größeren Mengen an Isobuten im Austrag, wie beispielsweise bei Einsatz von FCC-C₄-Fraktionen oder bei der Abtrennung von Isobuten durch sauerkatalysierte Polymerisation zu Polyisobuten (Teilumsatz), kann der verbleibende Raffinatstrom gemäß einer Ausführungsform der Erfindung vor der Weiterverarbeitung destillativ aufbereitet werden.

### Reinigung des Raffinat II-Stroms an Adsorbermaterialien

Der nach der Veretherung/Polymerisation (bzw. Destillation) erhaltene Raffinat II-Strom wird an mindestens einem guard bed, bestehend aus hochoberflächigen Aluminiumoxiden, Kieselgelen, Alumsolikaten oder Molsieben, gereinigt. Das Schutzbett dient hierbei zum Trocknen des C₄-Stroms sowie zur Entfernung von Substanzen, welche als Katalysatorgift im nachfolgenden Metatheseschritt wirken können. Die bevorzugten Adsorbermaterialien sind Selexsorb CD und CDO sowie 3Ä- und NaX-Molsiebe (13X). Die Reinigung erfolgt in Trockentürmen bei Temperaturen und Drucken, die so gewählt sind, daß sämtliche Komponenten in der flüssigen Phase vorliegen. Gegebenenfalls wird der Reinigungsschritt zur Feed-Vorwärmung für den nachfolgenden Metatheseschritt eingesetzt.

Der verbleibende Raffinat II-Strom ist annähernd frei von Wasser, Oxygenaten, organischen Chloriden und Schwefelverbindungen.

Bei Durchführung des Veretherungsschritts mit Methanol zur Herstellung von MTBE kann es aufgrund der Bildung von Dimethylether als Nebenkomponente erforderlich sein, mehrere Reinigungsschritte zu kombinieren bzw. hintereinander zu schalten.

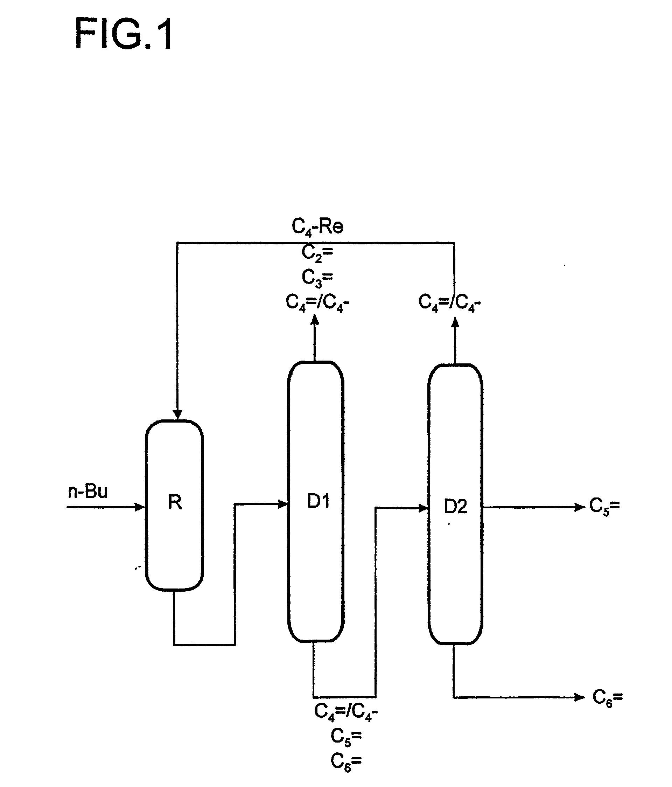

Zur Maximierung der Ausbeute an 2-Penten und 3-Hexen werden im erfindungsgemäßen Verfahren folgende Varianten, welche nachfolgend in der Zeichnung in Fig. 1, Fig. 2 und Fig. 3 anhand vereinfachter Verfahrensschemata erläutert werden, bevorzugt. Die Umsetzungen sind zur besseren Übersicht jeweils ohne signifikante Mengen Isobuten im C₄-Feed dargestellt. Dabei bedeuten:
- C₂⁼: = Ethen
- C₃⁼: = Propen
- C₄⁼: = 1- und 2-Buten
- C₄⁼: = n- und i-Butan
- C₅⁼: = 2-Penten
- C₆⁼: = 3-Hexen
- C₄-Re: = C4-Recycle
- n-Bu: = n-Butene
- C_{4/5}-Re: = C_{4/5}-Recycle
- C₅-Re: = C₅-Recycle

### • Metathese mit zweistufiger Destillation und partieller C₄-Rückführung Gewinnung von 2-Penten und 3-Hexen (Fig. 1)

Der aus C₂-C₆-Olefinen und Butanen bestehende Austragsstrom des Metathesereaktors R wird in der Destillation D1 getrennt in eine Fraktion aus Ethen, Propen und 0 bis 50 % der nicht umgesetzten Butene und Butane, welche gegebenenfalls in die Aufarbeitungssequenz eines Crackers eingespeist werden, sowie eine Hochsiederfraktion, die aus Rest-C₄ sowie gebildetem 2-Penten und 3-Hexen besteht. Letztere wird in einer Kolonne D2 destilliert zur Gewinnung von 2-Penten als Seitenabzug und 3-Hexen. Beide Ströme fallen mit einer Reinheit von > 99 % an. Die C₄-Fraktion wird über Kopf abgezogen und in den Metathesereaktor R zurückgeführt. Kolonne D2 kann auch als Trennblechkolonne ausgelegt sein. Der Reaktor R und die Kolonne D1 können zu einer Reaktivdestillationseinheit gekoppelt werden.

Der Kopfaustrag der Destillationskolonne D1 kann zur Erhöhung der Ausbeute an C₅/C₆-Olefinen bei Bedarf in den Metathesereaktor R zurückgeführt werden.

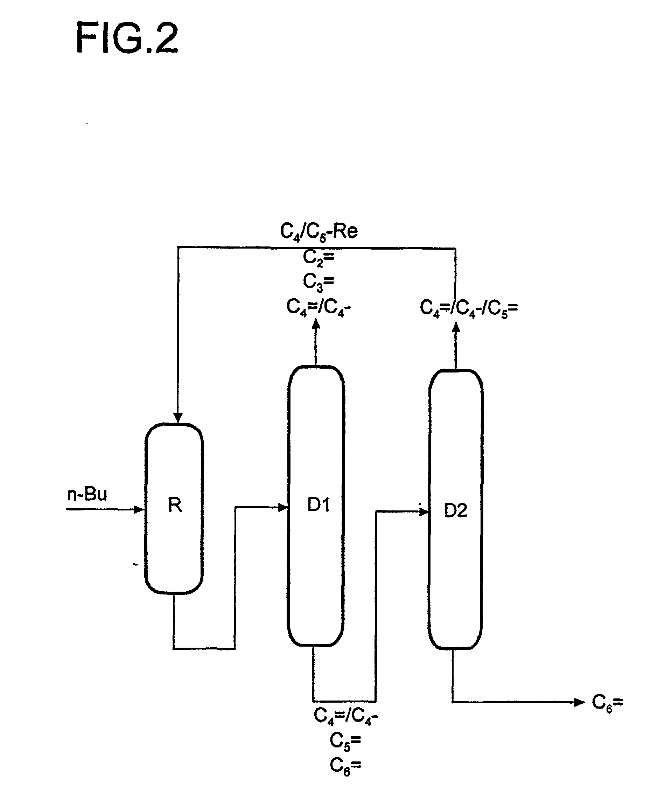

### • Metatheseschritt mit zweistufiger Destillation und partieller C₄- und C₅-Rückführung Maximierung der Hexenausbeute (Fig. 2)

Der aus C₂-C₆-Olefinen und Butanen bestehende Austragsstrom des Metathesereaktors R wird in der Destillation D1 getrennt in eine Fraktion aus Ethen, Propen und 0 bis 50 % der nicht umgesetzten Butene und Butane, welche gegebenenfalls in die Aufarbeitungssequenz eines Crackers eingespeist werden, sowie einer Hochsiederfraktion, die aus Rest-C₄ sowie gebildetem 2-Penten und 3-Hexen besteht. Letztere wird in einer Kolonne D2 destilliert zur Gewinnung von 3-Hexen, welches mit einer Reinheit von > 99 % isoliert wird. Die C₄-Fraktion wird zusammen mit Penten über Kopf genommen und in den Metathesereaktor R zurückgeführt. Der Reaktor R und die Kolonnen D1 und D2 können zu einer Reaktivdestillationseinheit gekoppelt werden.

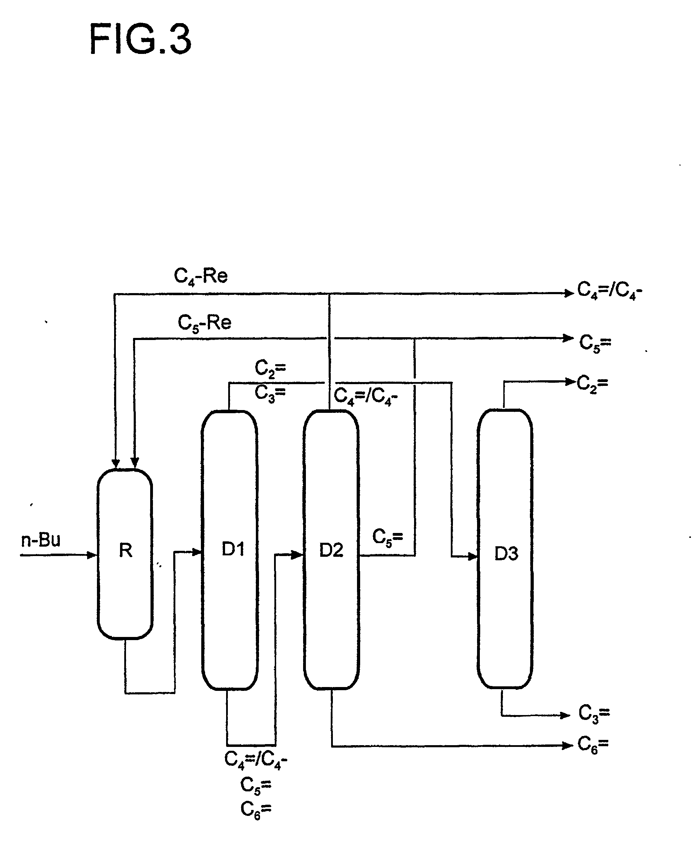

### • Metatheseschritt mit dreistufiger Destillation/partielle C₄- und C₅-Rückführung zur Maximierung der Hexenausbeute (Fig. 3)

Der aus C₂-C₆-Olefinen und Butanen bestehende Austragsstrom des Metathesereaktors R wird in der Destillation D1 getrennt in eine aus Ethen und Propen bestehende Leichtsiederfraktion, welche entweder in die Aufarbeitungssequenz eines Crackers eingespeist werden kann oder vorzugsweise in einer weiteren Destillationskolonne D3 in die Reinkomponenten Ethen und Propen getrennt wird, sowie in eine Hochsiederfraktion, die aus C₄-Olefinen und Butanen sowie gebildetem 2-Penten und 3-Hexen besteht. Letztere wird in einer Kolonne D2, die ggf. als Seitenabzugskolonne oder Trennwandkolonne ausgelegt sein kann, getrennt in eine aus C₄-Olefinen und Butanen bestehende Leichtsiederfraktion, die ganz oder teilweise in den Metatheseschritt zurückgeführt werden kann, in eine vorzugsweise aus 2-Penten bestehende Mittelsiederfraktion, die ganz oder teilweise in den Metatheseschritt zurückgeführt werden kann, und in eine aus 3-Hexen (Reinheit von > 99 %) bestehende Wertprodukt-Hochsiederfraktion, die ausgeschleust wird.

Als Katalysatoren werden literaturbekannte heterogene Rhenium-Katalysatoren, wie Re₂O₇ auf γ-Al₂O₃ oder auf Mischträgern, wie z.B. SiO₂/Al₂O₃, B₂O₃/SiO₂/Al₂O₃ oder Fe₂O₃/Al₂O₃ mit unterschiedlichem Metallgehalt bevorzugt. Der Rheniumoxid-Gehalt beträgt unabhängig vom gewählten Träger zwischen 1 und 20 %, vorzugsweise zwischen 3 und 10 %.
Die Katalysatoren werden frisch calziniert eingesetzt und bedürfen keiner weiteren Aktivierung (z.B. durch Alkylierungsmittel). Desaktivierter Katalysator kann durch Abbrennen von Coke-Rückständen bei Temperaturen oberhalb von 400°C im Luftstrom und Abkühlung unter Inertgas-Atmosphäre mehrfach regeneriert werden.

Weniger geeignet, aber dennoch erfindungsgemäß einsetzbar sind homogene Katalysatoren, die zwar teilweise aktiver sind, jedoch eine vergleichsweise deutlich geringere Standzeit aufweisen:

K.J. Ivin, J. Organomet. Catal. A: Chemical **1998**, 133, 1-16; K.J. Ivin, I.C. Mol, Olefin Metathesis and Metathesis Polymerization, 2^{nd} edition, Academic Press, New York, **1996**; G.W. Parshall, S.D. Ittel, Homogeneous, Catalysis, 2. Aufl., **1992**, John Wiley & Sons, New York, Chichester, Brisbane, Toronto, Singapur, S. 217ff; R.H. Grubbs in Prog. Inorg. Chem., S. Lippard (Hrsg.), John Wiley & Sons, New York, **1978**, Vol. 24, 1-50; R.H. Grubbs in Comprehensive Organomet. Chem., G. Wilkinson (Hrsg.), Pergamon Press, Ltd., New York, **1982**, Vol. 8, 499-551; D.S. Breslow, Prog. Polym. Sci. **1993**, Vol. 18, 1141-1195] wie auch protischem Medium und an Luftsauerstoff stabile homogene Metathesekatalysatoren, wie die von R.H. Grubbs et al. in WO 93/20111, WO 96/04289, WO 96/06185, WO 97/03096 und WO 98/21214 beschriebenen definierten Ruthenium-Alkylidenverbindungen der allgemeinen Zusammensetzung RuX₂(=CHR)(PR'₃)₂ (R=R'= Alkyl, Aryl) sowie _{"}in situ" aus [Ru(n⁶-Aryl)X₂]₂, Phosphanen PR₃ und Diazoverbindungen RCHN₂ erzeugte Gemische, über deren Eignung als Metathesekatalysator A.F. Noels in J. Chem. Soc., Chem. Commun. **1995**, 1127-1128 berichtete.

Demgegenüber erweisen sich heterogene Katalysatoren, insbesondere Molybdän-Wolfram- und Rheniumoxide auf anorganischen oxiden Trägern, die gegebenenfalls mit Alkylierungsmitteln vorbehandelt werden, häufig als empfindlicher gegenüber Verunreinigungen im Feed. Ihr Vorteil gegenüber Homogenkatalysatoren mit höherer Aktivität liegt in der sehr einfachen Katalysator-Regeneration, die üblicherweise durch Abbrennen von Coke-Rückständen im Luftstrom erfolgt. Ein Vergleich der Heterogenkontakte untereinander zeigt, daß Re₂O₇/Al₂O₃ bereits unter sehr milden Reaktionsbedingungen (T = 20 bis 80°C) aktiv ist, während MO₃/SiO₂ (M = Mo, W) erst bei Temperaturen oberhalb von 100 bis 150°C Aktivität entwickelt und demzufolge als Nebenreaktionen C=C-Doppelbindungsisomerisierung auftreten kann.

Ferner sind zu nennen:
- WO₃/SiO₂, präpariert aus (C₅H₅)W(CO)₃Cl und SiO₂ in J. Mol Catal. **1995**, 95, 75-83;
- 3-Komponenten-System, bestehend aus [Mo(NO)₂(OR)₂]n, SnEt₄ und AlCl₃ in J. Mol. Catal. **1991**, 64, 171-178 und J. Mol. Catal 1989, 57, 207-220;
- Nitridomolybdän (VI)-Komplexe aus hochaktive Präkatalysatoren in J. Organomet. Chem. **1982**, 229, C₁₉-C₂₃;
- heterogene SiO₂-geträgerte MoO₃ und WO₃-Katalysatoren in J. Chem. Soc., Faraday Trans. /**1982**,78,2583-2592;
- geträgerte Mo-Katalysatoren in J. Chem. Soc., Faraday Trans. / **1981**, 77, 1763-1777;
- aktive Wolfram-Katalysatorvorstufe in J. Am. Chem. Soc. **1980**, 102(21), 6572-6574;
- Acetonitril(pentacarbonyl)wolfram in J. Catal. **1975**, 38, 482-484;
- Trichloro(nitrosyl)molybdän(II) als Katalysator-Vorstufe in Z. Chem. **1974**, 14, 284-285;
- W(CO)₅PPH₃/EtAlCl₂ in J. Catal. **1974**, 34, 196-202;
- WCl₆/n-BuLi in J. Catal **1973**, 28, 300-303;
- WCl₆/n-BuLi in J. Catal. **1972**, 26, 455-458;

FR 2 726 563 O₃ReO[Al(OR)(L)xO]nReO₃ mit R = C₁-C₄₀-Kohlenwasserstoff, n = 1-10, x = 0 oder 1 und L = Solvens,
EP-A-0 191 675, EP-A-0 129 474, BE 899897 Katalysatorsysteme aus Wolfram, 2 substituierten Phenolatresten und 4 anderen Liganden, u.a. einer Halogen-, Alkyl- bzw. Carbengruppe.
FR 2 499 083 Katalysatorsystem aus einem Wolfram-, Molybdän- oder Rhenium-Oxo-Übergangsmetallkomplex mit einer Lewissäure.

US 4,060,468 Katalysatorsystem aus einem Wolframsalz, einer sauerstoffhaltigen aromatischen Verbindung, z.B. 2,6-Dichlorphenol und wahlweise molekularem Sauerstoff.

BE 776,564 Katalysatorsystem aus einem Übergangsmetallsalz, einer metallorganischen Verbindung und einem Amin.

Für die Verbesserung der Cyclusdauer der eingesetzten Katalysatoren, vor allem der geträgerten Katalysatoren, empfiehlt sich der Einsatz einer Feed-Reinigung an Adsorberbetten (guard beds). Das Schutzbett dient hierbei zum Trocknen des C4-Stroms sowie zur Entfernung von Substanzen, welches als Katalysatorgift im nachfolgenden Metatheseschritt wirken können. 8 Die bevorzugten Adsorbermaterialien sind Selexsorb CD und CDO sowie 3Å- und NaX-Molsiebe (13X). Die Reinigung erfolgt in Trockentürmen bei Temperaturen und Drucken, die bevorzugt so gewählt sind, daß sämtliche Komponenten in der flüssigen Phase vorliegen. Gegebenenfalls wird der Reinigungsschritt zur Feed-Vorwärmung für den nachfolgenden Metatheseschritt eingesetzt. Es kann von Vorteil sein, mehrere Reinigungsschritte miteinander zu kombinieren bzw. hintereinander zu schalten.

Druck und Temperatur im Metatheseschritt sind so gewählt, daß sämtliche Reaktionspartner in der flüssigen Phase vorliegen (üblicherweise = 0 bis 150°C, bevorzugt 20 bis 80°C; p = 2 bis 200 bar). Alternativ kann es aber von Vorteil sein, insbesondere bei Feedströmen mit höherem Isobutengehalt, die Umsetzung in der Gasphase durchzuführen und/oder einen Katalysator einzusetzen, der über eine geringere Acidität verfügt.

In der Regel ist die Umsetzung nach 1 s bis 1 h, vorzugsweise nach 30 s bis 30 min beendet. Sie kann kontinuierlich oder diskontinuierlich in Reaktoren, wie Druckgasgefäßen, Strömungsrohren oder Reaktivdestillationsvorrichtungen durchgeführt werden, wobei Strömungsrohre bevorzugt werden.

### Beispiele

### Beispiel 1

### Kontinuierlicher Versuch zur zweistufigen Selektivhydrierung von Roh-C₄-Schnitt

Roh-C₄-Schnitt mit einer Zusammensetzung von 43,7 % Butadien (incl. Butenin und Butin), 14,3 % 1-Buten, 7,8 % 2-Butene und 7,2 % n-Butan wird in einem kontinuierlich durchströmten Rohrreaktor an 0,3 % Pd/Al₂O₃-Heterogenkontakt bei einem Fischzulauf von 1 kg/h Roh-C₄-Schnitt und einem Kreislauf von 8,2 kg/h mit einer LHSV von 9,0 h⁻¹ bei einer Reaktoreingangstemperatur von 20°C mit 175 N1/h Wasserstoff umgesetzt. Bei einem Butadien-Umsatz von 95,2 % wurde in der ersten Stufe der Selektivhydrierung unter diesen Bedingungen eine Gesamt-Buten-Selektivität von 99,6 % sowie eine 1-Buten-Selektivität von 56,5 % erzielt.

Ein typischer Reaktionsaustrag aus der ersten Stufe der Selektivhydrierung, bestehend aus 0,61 % Butadien (incl. Butenin und Butin), 26,9 % 1-Buten, 14,9 2-Butene und 11,6 % n-Butan wird in einem kontinuierlich durchströmten Rohrreaktor an 0,3 % Pd/Al₂O₃-Heterogenkontakt (H0-13L) bei einem Fischzulauf von 2,2 kg/h Reaktionsaustrag der 1. Stufe und einem Kreislauf von 4,0 kg/h mit einer LHSV von 20 h⁻¹ bei einer Reaktoreingangstemperatur von 60°C und einer Reaktorausgangstemperatur von 70°C mit 16 Nl/h Wasserstoff umgesetzt. Bei einem Butadien-Umsatz von 99,2 % wurde unter diesen Bedingungen bei einem 1-Buten-Erhalt von 58,2 % ein Raffinat I-Strom erhalten, der einen Restgehalt von 48 ppm Butadien aufweist.

### Beispiel 2

### Kontinuierlicher Versuch zur Abtrennung von Isobuten durch Veretherung mit Isobutanol

In einer dreistufigen Reaktorkaskade wird ein geflutetes und mit saurem Ionenaustauscher bestücktes Festbett von oben nach unten mit Raffinat I und Isobutanol durchströmt, wobei das Verhältnis von Isobutanol zu Isobuten im Feed auf 1,2 eingestellt wurde. Die Reaktoreingangstemperatur liegt bei 40°C, die Reaktorausgangstemperatur bei 65°C und der Reaktionsdruck bei 8 bar. Der gemessene Isobuten-Umsatz nach der ersten Stufe liegt bei 85 %. Im zweiten, ähnlich dimensionierten Reaktor wird der Umsatz bei einer Reaktoreingangstemperatur von 40°C, einer Reaktorausgangstemperatur bei 50°C und einem Reaktionsdruck von 8 bar auf 95 % erhöht. Im dritten, deutlich größeren Reaktor wird der Gleichgewichtsumsatz bei einer Reaktoreingangstemperatur und Reaktorausgangstemperatur von jeweils 40°C und einem Reaktionsdruck von 8 bar eingestellt. Der unter diesen Bedingungen nach destillativer Abtrennung von Isobutyl-tert.-butylether verbleibende Raffinat-Strom weist einen Isobuten-Restgehalt von 0,7 % auf.

### Beispiel 3

### Kontinuierlicher Versuch zur einstufigen Metathese von Raffinat II

Nach Feedreinigung über ein Adsorberbett aus Molsieb 13X wird mit einem Massenstrom von 1300 g 1 h bei einer Verweilszeit von 3 min eine aus 43,5 % 1-Buten, 36,2 2-Buten, 2,0 % Isobuten und 18,3 % Butanen bestehende C₄-Fraktion bei 40°C und 10 bar (Flüssigphase) kontinuierlich über ein mit Re₂O₇/Al₂O₃-Heterogenkontakt bestücktes Strömungsrohr geleitet. Der Reaktionsaustrag wird in einer zweistufigen Destillationssequenz aufgetrennt, wobei in der ersten Kolonne bei 10 bar eine C₂/C₃/C₄-Leichtsiederphase, bestehend aus 1,2 % Ethen, 38,7 % Propen, 31,3 % Butenen, 2,9 % Isobuten und 25,9 % Butanen über Kopf genommen wird. Der aus 28,0 % Butenen, 1,3 % Isobuten, 20,4 % Butanen, 27,8 % 2-Penten und 21,9 % 3-Hexen bestehende Sumpf wird anschließend einer bei 2 bar betriebenen zweiten Kolonne zugeführt, in welcher die C₄/C₅-Leichtsiederfraktion über Kopf abgetrennt und komplett in die Metathesereaktion zurückgeführt wird. Die im Sumpf anfallende Hochsiederfraktion bestand zu 99,5 % aus 3-Hexen. Die Prozentangaben entsprechen jeweils den entsprechenden Massenanteilen. Die ermittelten Butenumsätze liegen bei 91 % bezüglich 1-Buten und 50 % bezüglich 2-Buten. Die ermittelten Raum-Zeit-Ausbeuten lagen im Mittel bei 700 g/l•h Propen bzw. 760 g/l•h 3-Hexen.

## Patentansprüche

1. Verfahren zur Herstellung von C₅-/C₆-Olefinen aus einem olefinischen, C₄-Kohlenwasserstoffe enthaltenden Ausgangsstrom, **dadurch gekennzeichnet, daß**
a) in Gegenwart eines Metathesekatalysators, der mindestens eine Verbindung eines Metalls der VI.b, VII.b oder VIII. Nebengruppe des Periodensystems der Elemente enthält, eine Metathesereaktion durchgeführt wird, im Rahmen derer im Ausgangsstrom enthaltenes 1-Buten, 2-Buten und Isobuten zu einem Gemisch aus C₂-C₆-Olefinen und Butanen umgesetzt werden,
b) der so erhaltene Austragsstrom zunächst destillativ getrennt wird in eine aus C₂-C₄-Olefinen und Butanen oder aus C₂-C₃-Olefinen bestehende Leichtsiederfraktion **A**, die ausgeschleust wird, sowie in eine aus C₄-C₆-Olefinen und Butanen bestehende Schwersiederfraktion,
c) die aus b) erhaltene Schwersiederfraktion anschließend destillativ in eine aus Butenen und Butanen bestehende Leichtsiederfraktion **B**, eine aus Penten und Methylbuten bestehende Mittelsiederfraktion **C** und in eine aus Hexen und Methylpenten bestehende Schwersiederfraktion **D** getrennt wird,
d) wobei die Fraktionen **B** und/oder **C** komplett oder teilweise in den Verfahrensschritt a) zurückgeführt werden und die Fraktion **D** als Produkt ausgeschleust wird.

2. Verfahren nach Anspruch 1, **dadurch gekennzeichnet, daß** eine C₄-Fraktion eingesetzt wird, wie sie beim Steam- oder FCC-Cracken oder bei der Dehydrierung von Butan anfällt.

3. Verfahren nach Anspruch 1 oder 2, **dadurch gekennzeichnet, daß** als C₄-Fraktion Raffinat I oder II eingesetzt wird, wobei der C₄-Strom vor der Metathesereaktion durch entsprechende Behandlung an Adsorber-Schutzbetten von störenden Verunreinigungen befreit wird.

4. Verfahren nach einem der Ansprüche 1 bis 3, **dadurch gekennzeichnet, daß** die Metathesereaktion in Gegenwart von heterogenen Metathesekatalysatoren durchgeführt wird, die aus der Klasse der auf anorganischen Trägern aufgebrachten Übergangsmetallverbindungen von Metallen der VI.b, VII.b oder VIII. Nebengruppe des Periodensystems der Elemente ausgewählt sind.

5. Verfahren nach Anspruch 4, **dadurch gekennzeichnet, daß** als Metathesekatalysator Rheniumoxid auf γ-Aluminiumoxid oder auf Al₂O₃/B₂O₃/SiO₂-Mischträgern eingesetzt wird.

6. Verfahren zur Herstellung von C₅-/C₆-Olefinen und Propen aus Steamcrackeroder Raffinerie-C₄-Strömen, umfassend die Teilschritte
(1) Abtrennung von Butadien und acetylenischen Verbindungen durch gegebenenfalls Extraktion von Butadien mit einem Butadien-selektiven Lösungsmittel und nachfolgend oder alternativ Selektivhydrierung von in Roh-C₄-Schnitt enthaltenen Butadienen und acetylenischen Verunreinigungen, um einen Reaktionsaustrag zu erhalten, der n-Butene und Isobuten und im wesentlichen keine Butadiene und acetylenischen Verbindungen enthält,
(2) Abtrennung von Isobuten durch Umsetzung des in der vorstehenden Stufe erhaltenen Reaktionsaustrags mit einem Alkohol in Gegenwart eines sauren Katalysators zu einem Ether, Abtrennung des Ethers und des Alkohols, die gleichzeitig oder nach der Veretherung erfolgen kann, um einen Reaktionsaustrag zu erhalten, der n-Butene und gegebenenfalls Oxygenat-Verunreinigungen enthält, wobei gebildeter Ether ausgetragen oder zur Reingewinnung von Isobuten rückgespalten werden kann und dem Veretherungsschritt ein Destillationsschritt zur Abtrennung von Isobuten nachfolgen kann, wobei gegebenenfalls auch eingeschleuste C₃-, i-C₄- sowie C₅-Kohlenwasserstoffe destillativ im Rahmen der Aufarbeitung des Ethers abgetrennt werden können,
(3) Abtrennen der Oxygenat-Verunreinigungen des Austrags der vorstehenden Schritte an entsprechend ausgewählten Adsorbermaterialien,
(4) Metathesereaktion des so erhaltenen Raffinat II-Stromes gemäß einem der Ansprüche 1 bis 5.

7. Verfahren nach Anspruch 6, **dadurch gekennzeichnet, daß** der Teilschritt Selektivhydrierung von in Roh-C₄-Schnitt enthaltenen Butadienen und acetylenischen Verunreinigungen zweistufig durchgeführt wird durch Inkontaktbringen des Roh-C₄-Schnittes in flüssiger Phase mit einem Katalysator, der mindestens ein Metall, ausgewählt aus der Gruppe Nickel, Palladium und Platin, auf einem Träger enthält, bei einer Temperatur von 20 bis 200°C, einem Druck von 1 bis 50 bar, einer Volumengeschwindigkeit von 0,5 bis 30 m³ Frischfeed pro m³ Katalysator pro Stunde und einem Verhältnis von Recycle zu Zustrom von 0 bis 30 mit einem Molverhältnis von Wasserstoff zu Diolefinen von 0,5 bis 50, um einen Reaktionsaustrag zu erhalten, in welchem neben Isobuten die n-Butene 1-Buten und 2-Buten in einem Molverhältnis von 2:1 bis 1:10, vorzugsweise von 2:1 bis 1:2, vorliegen und im wesentlichen keine Diolefine und acetylenischen Verbindungen enthalten sind.

8. Verfahren nach Anspruch 6 oder 7, **dadurch gekennzeichnet, daß** der Teilschritt Butadien-Extraktion aus Roh-C₄-Schnitt mit einem Butadien-selektiven Lösungsmittel ausgewählt aus Aceton, Furfural, Acetonitril, Dimethylacetamid, Dimethylformamid und N-Methylpyrrolidon, durchgeführt wird, um einen Reaktionsaustrag zu erhalten, in welchem die n-Butene 1-Buten und 2-Buten in einem Molverhältnis von 2:1 bis 1:10, vorzugsweise von 2:1 bis 1:2 vorliegen.

9. Verfahren nach einem der Ansprüche 1 bis 8, **dadurch gekennzeichnet, daß** der Teilschritt Isobuten-Veretherung in einer dreistufigen Reaktorkaskade mit Methanol oder Isobutanol in Gegenwart eines sauren Ionenaustauschers durchgeführt wird, in der geflutete Festbettkatalysatoren von oben nach unten durchströmt werden, wobei die Reaktor-Eingangstemperatur 0 bis 60°C, die Ausgangstemperatur 25 bis 85°C, der Druck 2 bis 50 bar und das Verhältnis von Isobutanol zu Isobuten 0,8 bis 2,0 beträgt sowie der Gesamtumsatz dem Gleichgewichtsumsatz entspricht.

10. Verfahren nach einem der Ansprüche 1 bis 8, **dadurch gekennzeichnet, daß** der Teilschritt Isobuten-Abtrennung durch Oligomerisierung oder Polymerisation von Isobuten ausgehend von dem nach den vorstehend beschriebenen Stufen Butadien-Extraktion und/oder Selektivhydrierung erhaltenen Reaktionsaustrag in Gegenwart eines Katalysators verläuft, welcher ausgewählt ist aus heterogenen Kontakten, die ein Oxid eines Metalls der VI.b Nebengruppe des Periodensystems der Elemente auf einem sauren anorganischen Träger enthalten, um so einen Strom zu erzeugen, welcher weniger als 15 % Isobuten-Restgehalt aufweist.

11. Verfahren gemäß Anspruch 3 oder 6, **dadurch gekennzeichnet, daß** der Teilschritt Feedreinigung an mindestens einem Guard Bed, bestehend aus hochoberflächigen Aluminiumoxiden, Kieselgelen, Alumosilikaten oder Molsieben, durchgeführt wird.

## Claims

1. A process for preparing C₅-/C₆-olefins from an olefinic starting stream comprising C₄-hydrocarbons, which comprises
a) carrying out a metathesis reaction in the presence of a metathesis catalyst comprising at least one compound of a metal of transition group VIb, VIIb or VIII of the Periodic Table of the Elements so as to convert the 1-butene, 2-butene and isobutene present in the starting stream into a mixture of C₂-C₆-olefins and butanes,
b) firstly fractionating the resulting product stream by distillation to give a low boiler fraction A comprising C₂-C₄-olefins and butanes or C₂-C₃-olefins, which is discharged, and a high boiler fraction comprising C₄-C₆-olefins and butanes,
c) subsequently fractionating the high boiler fraction from b) by distillation to give a low boiler fraction B comprising butenes and butanes, an intermediate boiler fraction C comprising pentene and methylbutene and a high boiler fraction D comprising hexene and methylpentene,
d) where all or part of the fractions B and/or C are recirculated to the process step a) and the fraction D is discharged as product.

2. A process as claimed in claim 1, wherein the C₄ fraction is obtained from a steam cracker or FCC plant or in the dehydrogenation of butane.

3. A process as claimed in claim 1 or 2, wherein the C₄ fraction used is raffinate I or II and is freed of interfering impurities by appropriate treatment over adsorbent guard beds prior to the metathesis reaction.

4. A process as claimed in any of claims 1 to 3, wherein the metathesis reaction is carried out in the presence of heterogeneous metathesis catalysts selected from the class consisting of transition metal compounds of metals of transition groups VIb, VIIb and VIII of the Periodic Table of the Elements applied to inorganic supports.

5. A process as claimed in claim 4, wherein the metathesis catalyst used is rhenium oxide on γ-aluminum oxide or on Al₂O₃/B₂O₃/SiO₂ mixed supports.

6. A process for preparing C₅/C₆-olefins and propene from steam cracker or refinery C₄ streams, comprising the substeps
(1) removal of butadiene and acetylenic compounds by, if desired, extracting butadiene with a butadiene-selective solvent and subsequently or alternatively selectively hydrogenating butadienes and acetylenic impurities present in the crude C₄ fraction to give a reaction product comprising n-butenes and isobutene and essentially no butadienes and acetylenic compounds,
(2) removal of isobutene by reacting the reaction product from the preceding stage with an alcohol in the presence of an acid catalyst to give an ether, and separating off the ether and the alcohol either simultaneously with or after the etherification to give a reaction product comprising n-butenes and possibly oxygen-containing impurities, where the ether formed can be discharged or redissociated to obtain pure isobutene and the etherification step can be followed by a distillation step for separating off isobutene, where, if desired, introduced C₃-, i-C₄- and C₅-hydrocarbons can be removed by distillation in the work-up of the ether,
(3) removal of the oxygen-containing impurities from the product of the preceding steps over appropriately selected adsorber materials,
(4) metathesis of the resulting raffinate II stream as claimed in any of claims 1 to 5.

7. A process as claimed in claim 6, wherein the substep of selective hydrogenation of butadienes and acetylenic impurities present in the crude C₄ fraction is carried out in two stages by bringing the crude C₄ fraction into contact with a catalyst comprising at least one metal selected from the group consisting of nickel, palladium and platinum on a support, at from 20 to 200°C, a pressure of from 1 to 50 bar, a volume flow of from 0.5 to 30 m³ of fresh feed per m³ of catalyst per hour and a ratio of recycle to feed stream of from 0 to 30 at a molar ratio of hydrogen to diolefins of from 0.5 to 50 to give a reaction product in which, apart from isobutene, the n-butenes 1-butene and 2-butene are present in a molar ratio of from 2:1 to 1:10, preferably from 2:1 to 1:2, and in which essentially no diolefins and acetylenic compounds are present.

8. A process as claimed in claim 6 or 7, wherein the substep of butadiene extraction from crude C₄ fraction is carried out using a butadiene-selective solvent selected from among acetone, furfural, acetonitrile, dimethylacetamide, dimethylformamide and N-methylpyrrolidone to give a reaction product in which the n-butenes 1-butene and 2-butene are present in a molar ratio of from 2:1 to 1:10, preferably from 2:1 to 1:2.

9. A process as claimed in any of claims 1 to 8, wherein the substep of isobutene etherification is carried out in a three-stage reactor cascade using methanol or isobutanol in the presence of an acid ion exchanger, in which the extraction mixture flows from the top downward through the flooded fixed-bed catalyst, where the reactor inlet temperature is from 0 to 60°C, the outlet temperature is from 25 to 85°C, the pressure is from 2 to 50 bar and the ratio of isobutanol to isobutene is from 0.8 to 2.0, and the total conversion corresponds to the equilibrium conversion.

10. A process as claimed in any of claims 1 to 8, wherein the substep of isobutene removal proceeds by oligomerization or polymerization of isobutene starting from the reaction product obtained from the above-described step of butadiene extraction and/or selective hydrogenation and in the presence of a catalyst selected from the group consisting of heterogeneous catalysts comprising an oxide of a metal of transition group VIb of the Periodic Table of the Elements on an acidic inorganic support to produce a stream having a residual isobutene content of less than 15%.

11. A process as claimed in claim 3 or 6, wherein the substep of feed purification is carried out over at least one guard bed comprising high surface area aluminum oxides, silica gels, aluminosilicates or molecular sieves.

## Revendications

1. Procédé de préparation d'oléfines en C₅/C₆ à partir d'un courant de départ oléfinique contenant des hydrocarbures en C₄, **caractérisé en ce que**
a) en présence d'un catalyseur de métathèse, qui contient au moins un composé d'un métal du groupe secondaire VI.b, VII.b ou VIII du Tableau Périodique des Eléments, on met en oeuvre une réaction de métathèse, dans le cadre de laquelle on fait réagir, pour obtenir un mélange d'oléfines en C₂-C₆ et de butanes, le 1-butène, le 2-butène et l'isobutène contenus dans le courant de départ,
b) le courant de sortie ainsi obtenu est d'abord séparé par distillation en une fraction légère A, constituée d'oléfines en C₂-C₄ et de butanes ou d'oléfines en C₂-C₃, fraction qui est évacuée, et en une fraction lourde, constituée d'oléfines en C₄-C₆ et de butanes,
c) la fraction lourde obtenue en b) est ensuite séparée par distillation en une fraction légère constituée de butènes et de butanes, en une fraction moyenne C constituée de pentène et de méthylpentène, et en une fraction lourde D constituée d'hexène et de méthylpentène,
d) les fractions B et/ou C étant en totalité ou en partie renvoyées dans l'étape a) du procédé, la fraction D étant évacuée sous forme d'un produit.

2. Procédé selon la revendication 1, **caractérisé en ce qu'**on utilise une fraction en C₄ telle celle qui est obtenue lors du vapocraquage ou du craquage catalytique fluide FCC, ou lors de la déshydrogénation du butane.

3. Procédé selon la revendication 1 ou 2, **caractérisé en ce qu'**on utilise en tant que fraction en C₄ un raffinat I ou II, ce à l'occasion de quoi le courant en C₄ est, avant la réaction de métathèse, débarrassée des impuretés perturbatrices par un traitement approprié sur des lits protecteurs adsorbants.

4. Procédé selon l'une des revendications 1 à 3, **caractérisé en ce que** la réaction de métathèse est mise en oeuvre en présence de catalyseurs de métathèse hétérogènes, qui sont choisis dans la classe des composés de métaux de transition, appliqués sur des supports organiques, de métaux du groupe VI.b, VII.b ou VIII du Tableau Périodique des Eléments.

5. Procédé selon la revendication 4, **caractérisé en ce qu'**on utilise en tant que catalyseur de métathèse de l'oxyde de rhénium sur de l'oxyde d'aluminium y ou sur des supports mixtes Al₂O₃/B₂O₃/SiO₂.

6. Procédé de préparation d'oléfines en C₅/C₆ et de propène à partir de courants de vapocraquage ou de raffinerie en C₄, comprenant les étapes partielles suivantes :
(1) séparation du butadiène et des composés acétyléniques, par extraction éventuelle du butadiène avec un solvant sélectif du butadiène, puis, ou en remplacement, par hydrogénation sélective de butadiènes contenus dans la fraction brute en C₄ et d'impuretés acétyléniques, pour obtenir un produit de réaction qui contient des n-butènes et de l'isobutène, et ne contient pour l'essentiel aucun butadiène ni aucun composé acétylénique,
(2) séparation de l'isobutène par réaction du produit de la réaction, obtenu dans l'étape précédente, avec un alcool en présence d'un catalyseur acide pour donner un éther, séparation de l'éther et de l'alcool, qui peut se faire en même temps que l'éthérification ou après l'éthérification, pour obtenir un produit de réaction qui contient des n-butènes et éventuellement des impuretés oxygénées, l'éther formé pouvant être évacué, ou encore redécomposé pour obtenir de l'isobutène pur, et l'étape d'éthérification peut être suivie d'une étape de distillation pour séparer l'isobutène, auquel cas éventuellement aussi on peut séparer par distillation, dans le cadre du traitement de l'éther, les hydrocarbures en C₃, en i-C₄ et en C₅ qui ont été introduits,
(3) séparation des impuretés oxygénées du produit des étapes ci-dessus, sur des matériaux adsorbants convenablement sélectionnés,
(4) réaction de métathèse du courant ainsi obtenu du raffinât II selon l'une des revendications 1 à 5.

7. Procédé selon la revendication 6, **caractérisé en ce que** l'étape partielle d'hydrogénation sélective de butadiènes et d'impuretés acétyléniques contenus dans la fraction brute en C₄ est mise en oeuvre en deux étapes, par mise en contact de la fraction brute en C₄ en phase liquide avec un catalyseur qui contient sur un support au moins un métal choisi dans l'ensemble comprenant le nickel, le palladium et le platine, à une température de 20 à 200°C, sous une pression de 1 à 50 bar, pour une vitesse spatiale horaire en volume de 0,5 à 30 m³ de charge fraîche par mètre cube de catalyseur par heure, et avec un rapport du recyclat au courant de charge de 0 à 30, avec un rapport en moles de l'hydrogène aux dioléfines de 0,5 à 50, pour obtenir un produit de réaction dans lequel sont présents, outre l'isobutène, les n-butènes 1-butène et 2-butène selon un rapport en moles de 2:1 à 1:10 et de préférence de 2:1 à 1:2, et dans lequel il n'y a pour ainsi dire aucune dioléfine, ni aucun composé acétylénique.

8. Procédé selon la revendication 6 ou 7, **caractérisé en ce que** l'étape partielle d'extraction du butadiène à partir de la fraction brute en C₄ est réalisée à l'aide d'un solvant sélectif du butadiène, choisi parmi l'acétone, le furfural, l'acétonitrile, le diméthylacétamide, le diméthylformamide et la N-méthylpyrrolidone, pour obtenir un produit de réaction dans lequel les n-butènes 1-butène et 2-butène sont présents selon un rapport en moles de 2:1 à 1:10 et de préférence de 2:1 à 1:2.

9. Procédé selon l'une des revendications 1 à 8, **caractérisé en ce que** l'étape partielle d'éthérification de l'isobutène est mise en oeuvre dans une cascade de réacteurs à trois étages avec du méthanol ou de l'isobutanol en présence d'un échangeur d'ions acide, où des catalyseurs noyés en lit fixe passent de haut en bas, la température d'entrée du réacteur étant de 0 à 60°C, la température de sortie étant de 25 à 85°C, la pression étant de 2 à 50 bar, et le rapport de l'isobutanol à l'isobutène étant de 0,8 à 2,0, le taux de conversion totale correspondant au taux de conversion à l'équilibre.

10. Procédé selon l'une des revendications 1 à 8, **caractérisé en ce que** l'étape partielle de séparation de l'isobutène s'effectue par oligomérisation ou polymérisation de l'isobutène à partir du produit de la réaction, obtenu après les étapes décrites ci-dessus d'extraction du butadiène et/ou d'hydrogénation sélective, en présence d'un catalyseur qui est choisi parmi les catalyseurs hétérogènes contenant un oxyde d'un métal du groupe secondaire VI.b du Tableau Périodique des Eléments sur un support inorganique acide, pour produire un courant qui présente une teneur résiduelle en isobutène inférieure à 15%.

11. Procédé selon la revendication 3 ou 6, **caractérisé en ce que** l'étape partielle de purification de la charge est réalisée sur au moins un lit de garde, constitué d'oxydes d'aluminium, de gels de silice, d'aluminosilicates ou de tamis moléculaires à grande aire spécifique.
